# EUROPEAN PATENT SPECIFICATION

(11) **EP 0 649 318 B1**
(45) Date of publication and mention of the grant of the patent: **24.03.1999**
(21) Application number: 94916186.3
(22) Date of filing: 03.05.1994
(51) Int. Cl.: A61M 5/315

(54) **SYRINGE FOR MEDICINAL PURPOSES**
SPRITZE FÜR MEDIZINISCHE ZWECKE
SERINGUE A USAGE MEDICAL

(30) Priority: 06.05.1993 DE 4314987; 14.09.1993 DE 4331137
(43) Date of publication of application: 26.04.1995
(60) Divisional application: 98116586.3
(73) Proprietor: Becton, Dickinson and Company, Franklin Lakes, New Jersey 07417-1880 (US)
(72) Inventor: VETTER, Helmut, D-88212 Ravensburg (DE); OTTO, Thomas, D-88267 Vogt (DE); FRASCH, Eugen, D-88094 Oberteuringen (DE); BITDINGER, Ralf, F-38320 Herbeys (FR)
(74) Representative: Selting, Günther, Dipl.-Ing.
(86) International application number: EP9401401
(87) International publication number: WO9426334

(56) References cited:
- EP-A- 0 409 134
- DE-A- 2 945 869
- US-A- 4 711 637
- US-A- 4 883 471
- US-A- 4 946 441

## Description

The invention pertains to a syringe for medicinal purposes, with a syringe barrel, one end of which is designed as an adapter for a cannula or a closure piece, e.g., in the form of a tip cap, and with a plunger arranged in the syringe barrel and movable via a piston rod.

Syringes of this type are increasingly being marketed, especially as ready-to-use syringes in prefilled form, for self-administration by the patient, as has long been the case, especially for regular administration of insulin in diabetics.

In the case of unskilled or infirm people, there is a risk that before application the plunger will accidentally be pulled out of the syringe barrel so that, as a rule, the product is no longer usable.

US-A-4 711 637 discloses a syringe having a barrel with an outwardly extending barrel projection at the proximal end thereof. A sheet member is connected with the barrel projection, said sheet member having a radially inwardly directed protrusion extending between the vanes of a plunger rod. If the plunger rod is rotated about the longitudinal axis of the plunger rod, a sloping edge portion of the protrusion digs into the outer edges of the vanes of the plunger, to cause retention of the plunger and the barrel. The sheet member connected with the barrel is not intended to serve as a plunger brake, but it has a similar function. The sheet member has foldable tabs which may be bent around the barrel projection for fixing the sheet member to the barrel.

It is an object of the present invention to provide a syringe having a plunger brake that may be readily mounted to the syringe barrel in a defined position.

The syringe of the present invention is defined by claim 1.

According to the invention, which is provided for syringes in which the end of the syringe barrel away from the adapter has an annular projection extending radially outward, the rest piece is formed by a flat slip-on piece consisting of two disks separated by an interval, wherein the two disks are connected at the edges and between them, form a receiving pocket for the annular projection of the syringe cylinder, open toward one edge, and wherein the disk away from the adapter is provided with a circular opening coaxial to the syringe barrel, the diameter of which is smaller than the internal diameter of the barrel, and the other disk has a recess, open toward the edge, for passage of the barrel.

The edge of the coaxial opening in the one disk forms the stopper for the plunger. This slide-on piece guarantees firm connection with the cylinder and can also be designed such that it simultaneously forms a finger rest. Since the piston rod extends through the opening into one disk, unintentional removal of the plunger brake is not possible, at least with the piston rod mounted in place.

The recess advantageously has an edge shaped like the arc of a circle, coaxial with the circular opening, which surrounds the syringe barrel by somewhat more than 180 degrees and expands toward the edge of the disk. In this way, the slip-on piece can be connected simply and nevertheless firmly to the syringe barrel.

To prevent the piston rod, once placed in the syringe barrel, from being able to be accidentally withdrawn, it is also provided that the wall of the circular opening is designed as a cone mantle surface tapering toward the needle adapter, and the piston rod has on its end facing the plunger a radially outwardly projecting annular flange, the outer mantle surface of which likewise tapers conically toward the stopper, wherein the opening and/or the annular flange is designed to be elastic, at least in its marginal area.

It was found to be especially advantageous if the annular flange is received in a form-fitting way by the circular opening.

In the following, the invention will be explained in greater detail, based on exemplified embodiments shown in the drawings, which show:
- Fig. 1: a syringe not belonging to the invention, in side view, with the plunger brake clipped on,
- Fig. 2: a perspective view of the plunger brake in accordance with Fig. 1,
- Fig. 3: an embodiment of the plunger brake of the present invention with syringe cylinder only partially represented, in partial figure a before and in partial figure b after placement, in each case in perspective view,
- Fig. 4: in partial figures a to d a plunger brake in cross section or top view, in each case shown with and without the syringe barrel,
- Fig. 5: an additional embodiment similar to Fig. 3, prior to insertion of the piston rod, and
- Fig. 6: the object in accordance with Fig. 5, but after insertion of the piston rod.

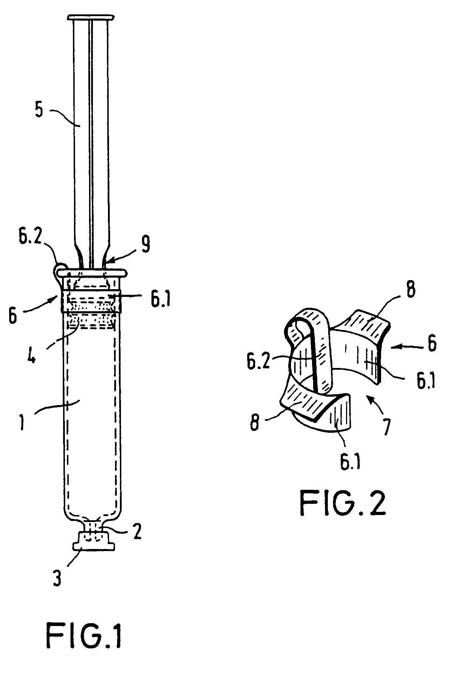

The embodiment of Figs. 1 and 2 is not in accordance with the present invention and serves only for explanatory purposes. The syringe for medicinal purposes shown in the drawing consists of a syringe barrel 1, one end of which is designed as an adapter 2 for a cannula or - as shown in Fig. 1 - for a closure piece 3 in the form of a tip cap. In the syringe barrel 1, a plunger 4 is arranged, which can be moved with the aid of a piston rod 5.

At the end of the syringe barrel 1 away from the cannula adapter 2, a plunger brake 6 is arranged. The plunger brake 6 consists, in detail, in a first embodiment shown in Figures 1 and 2, of a first annular piece 6.1, surrounding the barrel 1, and a finger 6.2 projecting into the interior of the barrel 1, connected to the annular piece 6.1. Here the free end of the finger 6.2 forms a stop for the plunger 4.

The plunger brake 6 prevents the plunger 4 from being accidentally pulled out of the barrel 1 in the case of incorrect use of the syringe, for example by a unskilled patient.

Also, during the manufacturing process of prefilled, ready-to-use syringes, the plunger brake 6 prevents, for example, the plunger 4 from being forced out of the barrel 1 due to excess pressure occurring in the interior during the post-sterilization of solutions previously filled into the barrel 1.

The annular portion 6.1 of the plunger brake 6, as shown in Fig. 2, consists of a separation slit 7 located on the side opposite the finger 6.2, as a result of which the plunger brake 6 can be clipped especially easily onto the syringe barrel 1 from the side. The annular portion 6.1 itself has an essentially sleeve-like shape, and is thus adapted in shape to the syringe barrel 1.

To improve handling, the annular piece 6.1 can be provided, in a way not shown in detail in the drawing, on its outer surface, with a riffling running in the circumferential direction. In addition, or alternatively, as shown in Fig. 2, the annular piece 6.1 can be provided with two radially extending, diametrically opposite wings 8, which form a finger rest. This is especially advantageous in the case of barrels with relatively small volume, since these generally do not have finger rests.

As Fig. 1 shows, the finger 6.2 lies against the inner wall of the syringe barrel 1, so that the piston rod 5 can be moved freely.

The piston rod 5 is tapered in the area 9 adjacent to the piston rod threads, so that the piston rod threads can be screwed into the plunger 4 without further efford, even when the plunger brake 6 is clipped into place.

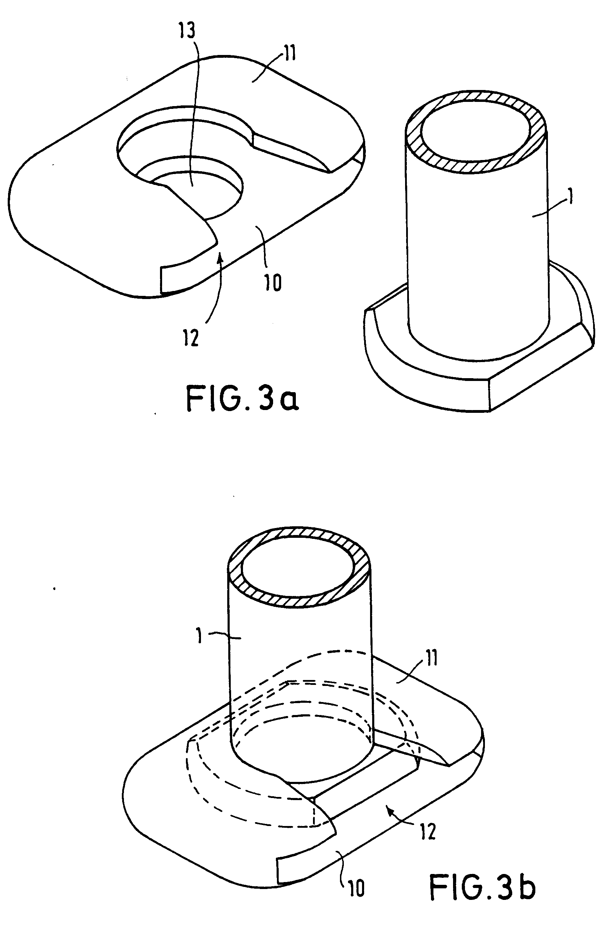

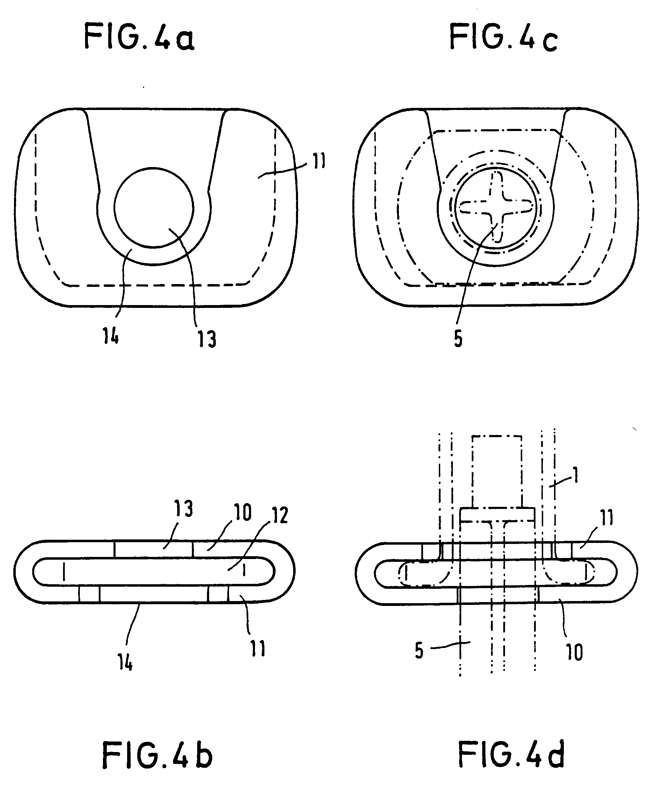

In the embodiment of the invention shown in Figures 3 and 4, the rest piece is formed by a flat slip-on piece made up of two disks 10,11 positioned at a distance from one another. The two disks 10,11 are connected at the edge, and between themselves form a receiving pocket 12, open toward one edge, for the radially outwardly extending edge of the syringe barrel 1.

The disk 10 away from the adapter 2 is provided with a circular opening 13 coaxial with the syringe barrel 1, the diameter of which opening is smaller than the internal diameter of the syringe barrel 1, so that the edge of this opening 13 forms the stop for the plunger 4. The other disk 11 has a recess 14, open toward the edge, for the syringe cylinder 1, so that this can be slid into the recess 14 from the side.

As is especially recognizable in Fig. 4, the recess 14 has an edge that is coaxial to the opening 13 and is shaped like the arc of a circle, surrounding the syringe barrel 1 by somewhat more than 180 degrees. In this way, firm retention of the slip-on piece on the syringe cylinder 1 is guaranteed. The recess 14 also broadens in the direction of the edge of the disk 11, simplifying the attachment of the slide-on piece to the syringe barrel 1.

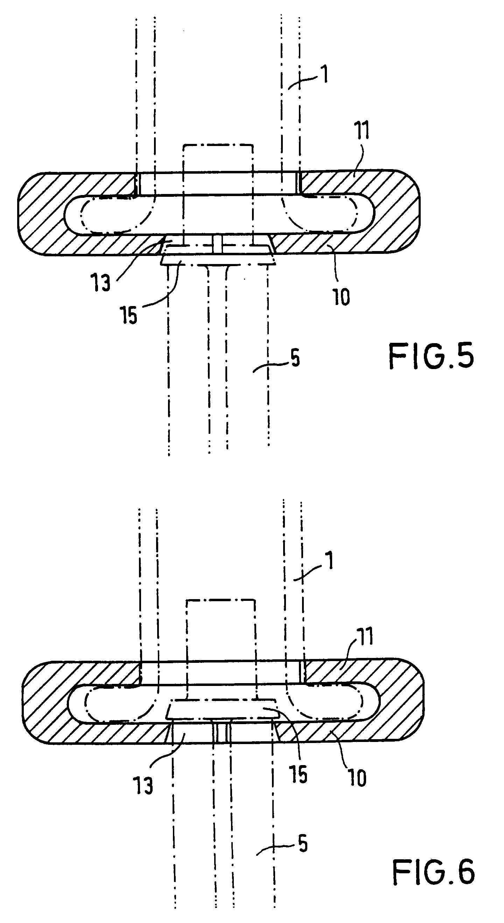

In addition, in order to prevent the piston rod from being able to be pulled out of the syringe barrel, the wall of the opening 13 - as shown in Figures 5 and 6 - is designed as a conical mantle surface tapering toward the adapter 2. The piston rod 5 has, at its end facing the plunger, a radially outwardly extending annular flange 15, the outer jacket surface of which likewise tapers conically, wherein the circular opening 13 and/or the annular flange 15 are designed to be elastic, at least in the edge area.

The dimensions are selected such that the annular flange 15 is received in a form-locking manner by the circular opening 13. In this way, to be sure, the piston rod can be slid into the syringe barrel 1, wherein the two conical surfaces of the circular opening 13 and the annular flange 15 slip past one another, but cannot be pulled out again, as is clearly apparent from Fig. 6.

## Claims

1. A syringe comprising an elongate hollow barrel having a distal end, an open proximal end and a chamber therebetween for retaining fluid, a tip at said distal end of said barrel having a passageway therethrough in fluid communication with said chamber, said barrel (1) including a radially outwardly extending barrel projection at said open proximal end, a plunger rod assembly (4,5) including a piston (4) in slidable fluid tight engagement inside said barrel (1) and an elongate plunger rod (5) connected to said piston (4) and extending proximally through said open end of said barrel, a plunger rod assembly brake removably attached to said open proximal end of said barrel (1) including a mounting member (10,11) partially surrounding and removably engaging said open proximal end of said barrel, a projection on said brake for engaging a complementary protrusion on said plunger rod assembly (4,5) so that proximal movement of said plunger rod assembly (4,5) with respect to said barrel (1) will cause said projection and said complementary protrusion to engage and prevent removal of said plunger rod assembly (4,5) from said barrel (1) during normal use of said syringe,
**characterized in that**
said mounting member (10,11) is a rest piece housing having a planar element (10) proximally spaced from a rest piece housing portion (11) and connected to said rest piece housing portion (11) to form a pocket (12) consisting of two disks separated by an interval and receiving said radially outwardly extending barrel projection, said planar element (10) including a circumferentially closed opening (13) therethrough, said brake projection being formed in said opening (13).

2. The syringe of claim 1 wherein said opening (13) is circular and said brake projection defines an inside diameter smaller than the inside diameter of said barrel (1).

3. The syringe of claim 2 wherein said brake projection defines a frusto-conically shaped inside diameter being smaller on the distal side of said planar element (10) than on said proximal side of said planar element (10).

4. The syringe of one of claims 1-3 wherein said complementary protrusion on said plunger rod (5) includes a disk-shaped structure substantially perpendicular to the longitudinal axis of said plunger rod (5).

5. The syringe of claim 4 wherein said disk-shaped structure has a tapered frusto-conically shaped out-side diameter being larger on the proximal side of said disk than on said distal side of said disk.

6. The syringe of one of claims 1-5 wherein the rest piece housing portion (11) has a recess (14), open toward the edge, said recess (14) being coaxial with the opening (13) and surrounding the barrel (1) somewhat more than 180°.

## Patentansprüche

1. Spritze mit einem länglichen hohlen Zylinder mit einem distalen Ende, einem offenen proximalen Ende und einer zwischen diesen angeordneten Kammer zum Halten von Fluid, einem an dem distalen Ende der Kammer angeordneten Endteil mit einem durchgehenden Durchlaß, der in Fluidverbindung mit der Kammer steht, wobei der Zylinder (1) an dem offenen proximalen Ende einen radial nach außen abstehenden Zylindervorsprung aufweist , einer Kolbenstangenvorrichtung (4,5), die einen Kolben (4), der in fluiddichtem Gleiteingriff innerhalb des Zylinders (1) angeordnet ist, und eine längliche Kolbenstange (5) aufweist, die mit dem Kolben (4) verbunden ist und proximal durch das offene Ende des Zylinders verläuft, und einer abnehmbar an dem offenen proximalen Ende des Zylinders (1) angeordneten Kolbenstangenvorrichtungs-Bremse mit einem Befestigungsteil (10,11), das das offene proximale Ende des Zylinders teilweise umgibt und abnehmbar damit zusammengreift, wobei ein an der Bremse ausgebildeter Vorsprung mit einem komplementären Vorsprung an der Kolbenstangenvorrichtung (4,5) derart zusammengreift, daß eine proximale Bewegung der Kolbenstangenvorrichtung (4,5) relativ zu dem Zylinder (1) ein Zusammengreifen des Vorsprungs und des komplementären Vorsprungs bewirkt und während der normalen Benutzung der Spritze ein Entfernen der Kolbenstangenvorrichtung (4,5) von dem Zylinder (1) verhindert,
**dadurch gekennzeichnet, daß**
das Befestigungsteil (10,11) ein Raststück-Gehäuse mit einem planaren Element (10) ist, das mit Abstand von einem Raststück-Gehäuseteil (11) proximal angeordnet und mit dem Raststück-Gehäuseteil (11) zur Bildung einer Tasche (12) verbunden ist, die aus zwei Scheiben besteht, welche durch einen Zwischenraum getrennt sind und den radial nach außen abstehenden Zylindervorsprung aufnehmen, wobei das planare Element (10) eine umfangsmäßig geschlossene Durchgangsöffnung (13) aufweist und wobei der Bremsvorsprung in der Öffnung (13) ausgebildet ist.

2. Spritze nach Anspruch 1, bei der die Öffnung (13) kreisförmig ist und der Bremsvorsprung einen Innendurchmesser definiert, der kleiner ist als der Innendurchmesser des Zylinders (1).

3. Spritze nach Anspruch 2, bei der der Bremsvorsprung einen kegelstumpfförmigen Innendurchmesserbereich definiert, der an der distalen Seite des planaren Elements (10) kleiner ist als an der proximalen Seite des planaren Elements (10).

4. Spritze nach einem der Ansprüche 1-3, bei der der komplementäre Vorsprung an der Kolbenstange (5) eine scheibenförmige Struktur aufweist, die im wesentlichen rechtwinklig zu der Längsachse der Kolbenstange (5) verläuft.

5. Spritze nach Anspruch 4, bei der die scheibenförmige Struktur einen sich verjüngenden kegelstumpfförmigen Außendurchmesserbereich definiert, der an der proximalen Seite der Scheibe kleiner ist als an der distalen Seite der Scheibe.

6. Spritze nach einem der Ansprüche 1-5, bei der das Raststück-Gehäuseteil (11) eine zum Rand hin offene Ausnehmung (14) aufweist, die koaxial mit der Öffnung (13) ist und den Zylinder (1) um etwas mehr als 180° umgibt.

## Revendications

1. Seringue comprenant un long corps cylindrique creux ayant une extrémité distale, une extrémité proximale ouverte et, entre elles, une chambre destinée à retenir un fluide, un embout situé à ladite extrémité distale dudit corps cylindrique et traversé par un passage placé en communication de transfert d'un fluide avec ladite chambre, ledit corps cylindrique (1) comportant une saillie s'étendant radialement vers l'extérieur à ladite extrémité proximale ouverte, un ensemble à piston et tige (4, 5) comportant un piston (4) étroitement ajusté à coulissement, avec étanchéité au fluide, à l'intérieur dudit corps cylindrique (1) et une longue tige de piston (5) reliée audit piston (4) et s'étendant du côté proximal à travers ladite extrémité ouverte dudit corps cylindrique, un frein d'ensemble à piston et tige fixé, de façon détachable, à ladite extrémité proximale ouverte dudit corps cylindrique (1) et comportant un élément de montage (10, 11) qui entoure pattiellement ladite extrémité proximale ouverte dudit corps cylindrique et vient en prise avec elle de façon détachable, une saillie formée sur ledit frein pour venir en prise avec une protubérance complémentaire formée sur ledit ensemble à piston et tige (4, 5) afin que le déplacement dudit ensemble à piston et tige (4, 5) vers le côté proximal, par rapport audit corps cylindrique (1), fasse venir en prise ladite saillie et ladite protubérance complémentaire et empêche de retirer ledit ensemble à piston et tige (4, 5) dudit corps cylindrique (1) au cours de l'utilisation normale de ladite seringue,
caractérisée en ce que
ledit élément de montage (10, 11) est un logement à pièce d'arrêt possédant un élément plan (10) placé à distance, côté proximal, d'une partie (11) du logement à pièce d'arrêt et relié à ladite partie (11) du logement à pièce d'arrêt de façon à former une poche (12) consistant en deux disques, séparés par un intervalle, et recevant ladite saillie, qui s'étend radialement vers l'extérieur, du corps cylindrique, ledit élément plan (10) comportant une ouverture (13) fermée sur son pourtour, qui le traverse, ladite saillie du frein étant formée dans ladite ouverture (13).

2. Seringue selon la revendication 1, dans laquelle ladite ouverture (13) est circulaire et ladite saillie du frein définit un diamètre intérieur plus petit que le diamètre intérieur dudit corps cylindrique (1).

3. Seringue selon la revendication 2, dans laquelle ladite saillie du frein définit un diamètre intérieur délimitant une forme tronconique, qui est plus petit sur la face distale dudit élément plan (10) que sur ladite face proximale dudit élément plan (10).

4. Seringue selon l'une des revendications 1 à 3, dans laquelle ladite protubérance complémentaire présente sur ladite tige de piston (5) comporte une structure en forme de disque, sensiblement perpendiculaire à l'axe longitudinal de ladite tige de piston (5).

5. Seringue selon la revendication 4, dans laquelle ladite structure en forme de disque présente un diamètre extérieur se rétrécissant sous une forme tronconique, qui est plus grand sur la face proximale dudit disque que sur ladite face distale dudit disque.

6. Seringue selon l'une des revendications 1 à 5, dans laquelle la partie (11) du logement à pièce d'arrêt présente un évidement (14), ouvert vers le bord ledit évidement (14) étant coaxial à l'ouverture (13) et entourant le corps cylindrique (1) sur un peu plus de 180°.
